# EUROPÄISCHE PATENTANMELDUNG

(11) **EP 1 241 294 A2**
(43) Veröffentlichungstag der Anmeldung: **18.09.2002**
(21) Anmeldenummer: 02005795.6
(22) Anmeldetag: 14.03.2002
(51) Int. Cl.: D06M 23/12, D06B 1/02

(54) **Fasermaterial sowie Verfahren und Behandlungsflüssigkeit zu seiner Herstellung**

(30) Priorität: 14.03.2001 DE 10112121
(71) Anmelder: Deotexis Inc., New York, N.Y. 10022-4838 (US)
(72) Erfinder: Tebbe, Gerold, 98000 Monte Carlo (MC)
(74) Vertreter: Ostertag, Reinhard

(57) **Zusammenfassung**

Die Erfindung betrifft ein gefaltetes Fasermaterial, z.B. eine Papier- oder Stoffserviette, das in gefaltetem Zustand zwischen zwei Lagen (12, 14, 16, 18) des Materials eingeschlossene Mikrokapseln aufweist, die einen Duftstoff oder eine Geruch neutralisierende Substanz (26) einschließen. Das Wandmaterial der Mikrokapseln ist kraftschlüssig gegebenenfalls durch ein Bindemittel mit benachbarten Materiallagen (12, 14, 16, 18) verbunden. Beim Entfalten des Fasermaterials werden die Mikrokapseln zerstört, und ein Duft oder eine Geruch neutralisierende Substanz wird freigesetzt.

## Beschreibung

Die Erfindung betrifft ein Fasermaterial gemäß dem Oberbegriff von Anspruch 1 sowie ein Verfahren zu dessen Herstellung.

Die Technik der Mikroverkapselung ist wohlbekannt. Dabei schlagen sich filmbildende Polymere auf dem einzuhüllenden Material nieder. Die mikroskopisch kleinen Kapseln lassen sich quasi zu einem Pulver trocknen. Auf diese Weise können z.B. Lebensmittelaromen und Parfums in eine Trockenmasse umgewandelt werden, die nicht eintrocknen kann und deren Inhalt bei Bedarf durch z.B. mechanische Einwirkung freigesetzt wird.

Auch die Mikroverkapselung von Duftstoffen ist bekannt. Beispielsweise sind Parfums in Mikrokapseln eingeschlossen worden, welche dann auf einer Werbeseite für das Parfum aufgebracht werden und durch Rubbeln mit einem harten Gegenstand das Parfum freisetzen können.

Bekannt sind auch Mikrokapseln für Duftstoffe, einen Geruch neutralisierende, pflegende oder heilende Substanzen, die auf der Haut zerrieben werden und dadurch den Inhalt freigeben. Dies bringt jedoch wie bei Sprays den Nachteil mit sich, daß die Duftstoffe nicht gleichmäßig über den ganzen Körper verteilt werden. Weiter ist eine solche herkömmliche Verwendung von Mikrokapseln in anderen Gegenständen, die normalerweise keiner Reibung unterzogen werden, unbequem.

Es wäre wünschenswert, über die Mikroverkapselung so zu verfügen, daß sie bei Aufnahme der Benutzung eines Gegenstandes einen Duftstoff oder eine einen schlechten Geruch neutralisierende Substanz freisetzt, ohne daß eine gesonderte Tätigkeit für diese Freisetzung erforderlich ist.

Diese Aufgabe der Erfindung wird durch ein Fasermaterial gemäß Anspruch 1 gelöst.

Wenn das gefaltete Fasermaterial nach Anspruch 1 entfaltet wird, zerbrechen die Mikrokapseln, wobei sie einen Duftstoff, eine Geruch neutralisierende, eine pflegende oder eine heilende Substanz freisetzen.

Anspruch 2 beschreibt eine vorteilhafte Verbindung zwischen Mikrokapseln und Fasermaterial.

Anspruch 3 sorgt dafür, daß die verbrauchten Mikrokapseln durch Auswaschen aus dem Fasermaterial entfernt werden können und daß das Material erneut mit Mikrokapseln beladen werden kann.

Anspruch 4 stellt sicher, daß das Auswaschen der verbrauchten Mikrokapseln und/oder des Bindemittels mit Wasser und ggf. Tensid vorgenommen werden kann.

Gemäß Anspruch 5 erhält man eine Serviette, die beim Entfalten automatisch einen Duftstoff freisetzt. Dies ist insbesondere für festliche Essen ein schöner Auftakt.

Die Anwendung gemäß Anspruch 6 hat den Vorteil, daß zugleich mit dem Entfalten eines Bekleidungsstückes ein Duft freigegeben wird. Dies verstärkt den Eindruck beim Benutzer, daß er ein neues sauberes Bekleidungsstück anzieht.

Die Verfahren nach Anspruch 7 bzw. 8 erlauben, ein Material auf sehr einfache Weise so zu präparieren, daß sich zwischen einzelnen Lagen des Fasermateriales Mikrokapseln befinden, die an beiden übereinandergelegten Lagen haften, so daß sie beim Auffalten des Fasermateriales aufgebrochen werden.

Bei Verwendung eines Mikrokapseln enthaltenden Fluids gemäß Anspruch 9 erhält man eine gute Bindung der Mikrokapseln zum Fasermaterial. Dadurch, daß die Duftstoff oder eine geruchsneutralisierende Substanz enthaltenden Mikrokapseln in dem Bindemittel dispergiert sind, erhält man eine bleibend gleichförmige Verteilung der Mikrokapseln im Bindemittel.

Gemäß Anspruch 10 kann auch ein Endbenutzer die Ausrüstung des Fasermateriales auf einfache Weise durchführen.

Bei dem erfindungsgemäßen Fasermaterial kann es sich beispielsweise um Papier, aber auch um Stoff handeln. Das Papier bildende Fasermaterial ist gewöhnlich ein Wegwerfgegenstand, der nur einmal benutzt wird, wie beispielsweise eine Papierserviette. Bei Fasermaterial, welches einen Stoff bildet, handelt es sich gewöhnlich um wiederverwendbare Gegenstände, wie beispielsweise Stoffservietten oder Bekleidungsstücke.

Das Innere der Mikrokapseln besteht gewöhnlich aus einer Flüssigkeit die einen Duftstoff freigibt oder eine geruchneutralisierende Wirkung aufweist. Es kann sich aber auch um ein Festkörpermaterial handeln, das adsorbiert eine Flüssigkeit oder ein Gas aufweist, das duftet oder einen Geruch neutralisiert, oder direkt verdampft (Sublimation). Der Kapselinhalt kann auch eine pflegende oder heilende Wirkung haben. In den Kapseln können auch Mischungen aus den vorgenannten Substanzen enthalten sein, oder die Mikrokapseln können ein Gemisch aus Mikrokapseln mit jeweils einem einzigen Inhhaltsstoff sein.

Durch Zerbrechen der Mikrokapseln wird deren Inhalt freigesetzt.

Die Wand der Mikrokapseln kann aus unterschiedlichen einerseits mit dem Fasermaterial direkt oder über ein Bindemittel verbindbaren andererseits bei mechanischer Belastung brechenden Materialien ausgewählt sein, wie z.B. Silikonelastomeren, Polyurethanen, Nitrilgummiarten, Chloroprengummiarten, Polyvinylalkoholen, Silikonen, Äthylen/Vinylactat-Polymeren, Acrylharzen und Harnstoff-Aldehyd-Polymer. Die Mikrokapseln können gemäß dem Verfahren von Beispiel 10 der US 3 516 941 A hergestellt werden.

Die Größen der Mikrokapseln betragen im allgemeinen etwa 2 - 300 µm, vorzugsweise etwa 4 - 80 µm und insbesondere etwa 15 - 25 µm. Durch die gewählte Mikrokapselgröße kann gesteuert werden, wie schwer bzw. leicht die Kapseln zerbrechen, wenn die beiden Fasermateriallagen, mit denen sie verbunden sind, auseinandergezogen werden.

Falls ein Bindemittel verwendet wird, um die Mikrokapseln mit den Faserlagen zu verbinden, kann dieses aus einer großen Vielfalt von Bindemitteln ausgewählt werden, wie Polyurethanen, Polyalcrylaten, Polyvinylharzen (z. B. Polyvinylalkohol, Polyvinylchlorid), Polyamiden, Polyestern, Polyolefinen, Stärken, Gummiarabicum, Gelatine, Cellulose und Silikonkautschuk. Für eine Anwendung bei textilen Materialien werden aus diesen Bindemitteln diejenigen bevorzugt, die in Wasser und ggf. Tensid löslich oder dispergierbar sind.

Insgesamt werden die beschriebenen Materialien so ausgewählt, daß der Eigenzusammenhalt der Mikrokapseln schwächer ist als die Haftung derselben an dem Fasermaterial bzw. dem Bindemittel und daß die Haftung des Bindemittels an dem Fasermaterial stärker ist als der Eigenzusammenhalt des Bindemittels.

Die Erfindung wird nachstehend unter Bezugnahme auf die Zeichnung anhand von Ausführungsbeispielen genauer erläutert. In der Zeichnung zeigen:
- Figur 1:: einen Schnitt durch einen Teil eines vierlagigen Materiales, bei dem zwischen den einzelnen Materiallagen Verbundschichten aus einem Bindemittel und Mikrokapseln, die einen Duftstoff enthalten, liegen, gezeigt in teilweise aufgefaltetem Zustand;
- Figur 2:: einen Schnitt durch ein zweilagiges Material, bei welchem Mikrokapseln zwischen mit den Materiallagen verbundenen Bindemittelschichten liegen; und
- Figur 3:: eine Sprayflasche, die eine Dispersion aus Mikrokugeln und Bindemittel enthält und zum Herstellen von Bindemittel/Mikrokapsel-Verbundschichten auf Fasermaterialien dient.

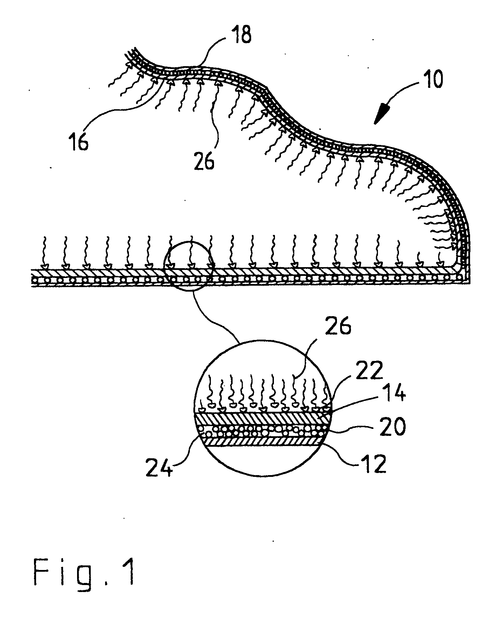

Figur 1 zeigt vier Lagen eines Fasermaterials, wobei jeweils zwei Lagen, nämlich die Lagen 12 und 14 und die Lagen 16 und 18, übereinander gefaltet sind. Zwischen den Lagen 12 und 14 bzw. 16 und 18 befinden sich Mikrokapseln 20, die kraftschlüssig mit beiden jeweils übereinanderliegenden Lagen verbunden sind.

Die Verbindung zwischen den Mikrokapseln und die Verbindung von Mikrokapseln mit dem Fasermaterial erfolgt bevorzugt durch ein Bindemittel 24. Die Mikrokapseln zwischen den Lagen 14 und 16 lagen ursprünglich ebenfalls in unversehrtem Zustand an beiden Lagen haftend vor. Durch Trennung der Lagen 14 und 16 sind die Mikrokapseln beschädigt worden, wodurch ihr Inhalt, ein Duftstoff oder eine Geruch neutralisierende Substanz freigesetzt worden ist wie durch gewellte Linien 26 angedeutet.

Bei dem oben beschriebenen Fasermaterial, welches z.B. eine zusammengefaltete Serviette darstellen kann, werden die in den Mikrokapseln 20 enthaltenen Duftstoffe, geruchsneutralisierenden, pflegenden oder heilenden Substanzen so lange zusammengehalten, bis die Faserlagen aufgefaltet werden. Dann wird flächig der Inhalt der Kapseln freigesetzt.

Bei den Faserlagen kann es sich auch um übereinandergelegte Lagen eines Kleidungsstückes handeln. So kann man z.B. nach dem Waschen Taschentücher kurz bevor man sie zusammenfaltet mit einer Flüssigkeit besprühen, die ein Bindemittel und hierin verteilt Duftstoff enthaltende Mikrokapseln aufweist. Das Bindemittel kann z.B. Reisstärke sein. Dieses Bindemittel wird beim nächsten Waschen des Taschentuches mit den Resten von Mikrokapseln herausgewaschen. Faltet man das Taschentuch nach dem Besprühen zusammen, so sind die Mikrokapseln über die getrocknete Reisstärke nun mit zwei übereinanderliegenden Lagen des Taschentuches verbunden. Bei Auffalten des Taschentuches werden die Mikrokapseln mechanisch zerstört, da ihr Kapselmaterial so gewählt ist, daß die mechanische Festigkeit der Mikrokapseln kleiner ist als die Stärke der Verbindung zwischen Mikrokapsel-Wandmaterial und dem Stoff des Taschentuches über die dazwischenliegende Reisstärke-Bindemittelschicht.

Ähnlich kann man auch andere Wäschestücke oder Kleidungsstücke mit einer Mikrokapseln enthaltenden Flüssigkeit besprühen, vorzugsweise durch Betätigen eines Sprays. Der beim Auffalten entstehende Duft gibt dem Benutzer ein verstärktes Gefühl dafür, daß das Wäsche- oder Bekleidungsstück, das er gerade in Benutzung nimmt, frisch gewaschen ist.

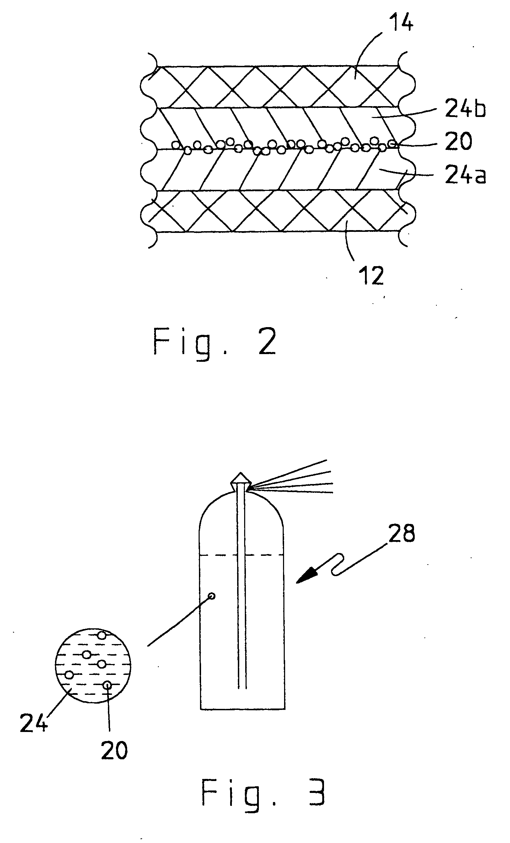

Bei dem abgewandelten Ausführungsbeispiel nach Figur 2 sind die Mikrokapseln 20 zwischen zwei Bindemittelschichten 24a, 24b eingeschlossen, die von der Lage 12 bzw. der Lage 14 getragen sind. Die Bindemittelschicht 24a und 24b waren zunächst eine durchgehende Bindemittelschicht, die auf eine durchgehende Materiallage aufgebracht (z.B. aufgesprüht) wurde, bevor die Lagen 12, 14 übereinandergelegt wurden. Auf diese ursprünglich einzige durchgehende Bindemittelschicht wurden bei ebenfalls noch nicht zusammengefalteten Lagen 12, 14 die Mikrokugeln 20 aufgebracht, z.B. aufgestäubt oder aufgesiebt.

Die ursprünglich durchgehende Lage mit der ursprünglich durchgehenden Bindemittelschicht und der darüber liegenden Schicht aus Mikrokapseln wurde dann gefaltet, wodurch die Mikrokapseln nun über die beiden Bindemittelschichten 24a, 24b mit den Lagen 12, 14 verbunden sind.

Zieht man die beiden Lagen 12, 14 auseinander, so stellt die nun innere Mikrokapselschicht eine Spaltfläche dar, und die Bindemittelschicht 24a und 24b trennen sich unter Zerstörung der Mikrokapseln 20. Damit wird ein Duftstoff oder eine geruchsneutralisierende Substanz freigegeben, die sich im Inneren der Mikrokapseln 20 befindet.

Bei dem in den Mikrokapseln eingeschlossenen Duftstoff oder geruchsneutralisierenden Material kann es sich auch um ein Festkörpermaterial handeln, welches nach der Zerstörung der Mikrokapselwand sublimiert.

Es versteht sich, daß man die übereinanderliegenden Lagen aus Fasermaterial über die gesamte Fläche hinweg mit Bindemittel und Mikrokapseln beschichten kann oder auch nur über Teilbereiche ihrer Kontaktfläche. Auf diese Weise kann man die Kraft, die notwendig ist, um die Lagen aufzufalten und die Intensität des beim Auffalten entstehenden Duftes regulieren.

Handelt es sich bei dem Fasermaterial um ein textiles Material, so wird vorzugsweise ein Bindemittel verwendet, das beim Waschen des textilen Materiales in Lösung geht. Auf diese Weise löst sich dann die an den Lagen noch hängenden Reste der Mikrokapselwände beim Waschen des textilen Materiales ab.

Falls gewünscht, kann man zusätzlich auch das Material der Mikrokapseln selbst so wählen, daß es beim Waschen in Lösung geht.

Für manche Anwendungsfälle reicht es auch aus, die Mikrokapseln unmittelbar nach ihrer Herstellung, wenn die Kapselwände noch klebrig sind, einfach auf die Oberfläche des Fasermateriales zu stäuben, bevor die Lagen des Fasermateriales übereinandergefaltet werden.

Bei Wäsche- oder Kleidungsstücken, die oft wiederverwendet werden, kann man dem Benutzer eine Ausrüst-Flüssigkeit zur Verfügung stellen, mit welcher er selbst Bindemittel-Mikrokapsel-Verbundschichten erzeugen kann, wie sie obenstehend unter Bezugnahme auf die Figuren 1 und 2 erläutert wurden.

Gemäß Figur 3 enthält eine Sprayflasche 28 eine wässrige Lösung aus einem Bindemittel (etwa Reisstärke) und Mikrokapseln 20. Die Sprayflasche 28 kann durch ein Treibgas unter Druck gesetzt sein oder einen Pumpmechanismus aufweisen, wie bei Sprayflaschen üblich.

## Patentansprüche

1. Gefaltetes Fasermaterial, **dadurch gekennzeichnet, daß** es in gefaltetem Zustand zwischen mindestens zwei Lagen des Materials verschlossene Mikrokapseln (20) aufweist, die einen Duftstoff, eine geruchneutralisierende, eine heilende, eine pflegende Substanz (23) oder Mischungen dieser Substanzen einschließen und deren Wandmaterial kraftschlüssig mit den benachbarten Materiallagen (12, 14; 16, 18) verbunden ist.

2. Fasermaterial nach Anspruch 1, **dadurch gekennzeichnet, daß** die Mikrokapseln (20) durch ein Bindemittel (24) mit den benachbarten Materiallagen (12, 14; 16, 18) verbunden sind.

3. Fasermaterial nach Anspruch 1 oder 2, **dadurch gekennzeichnet, daß** die Mikrokapseln (20, 22) auswaschbar sind.

4. Fasermaterial nach Anspruch 3, **dadurch gekennzeichnet, daß** das Wandmaterial der Mikrokapseln und/oder ein Bindemittel (24), durch welches die Mikrokapseln (20) mit dem Fasermaterial verbunden sind, in Wasser, zumindest in Waschmittel enthaltendem Wasser, löslich oder dispergierbar ist.

5. Fasermaterial nach einem der Ansprüche 1 bis 4, **dadurch gekennzeichnet, daß** es eine Serviette aus Papier oder Stoff bildet.

6. Fasermaterial nach einem der Ansprüche 1 bis 4,
**dadurch gekennzeichnet, daß** es ein Wäsche- oder Bekleidungsstück oder einen Teil eines solchen bildet.

7. Verfahren zur Herstellung eines Fasermaterials nach einem der Ansprüche 1 bis 6, **dadurch gekennzeichnet, daß** man (a) ein Fasermaterial im ungefalteten Zustand mit einem Mikrokapseln enthaltenden Fluid besprüht, (b) das Fasermaterial anschließend sofort so unter Anwendung von leichtem Druck faltet, daß zumindest ein Teil der Mikrokapseln an beiden so übereinander gelegten Lagen haften, (c) gegebenenfalls vorhandene Flüssigkeit verdunsten läßt.

8. Verfahren zur Herstellung eines Fasermateriales nach einem der Ansprüche 1 bis 6, **dadurch gekennzeichnet, daß** man (a) ein Fasermaterial in ungefaltetem Zustand mit einer Bindemittelschicht (24a, 24b) versieht, (b) auf das Bindemittel einen Duftstoff oder eine geruchsneutralisierende Substanz enthaltende Mikrokapseln (20) aufbringt und (c) das Fasermaterial unter Anwendung von leichtem Druck faltet, so daß zumindest ein Teil der Mikrokapseln an beiden übereinandergelegten Bindemittelschichten haftet.

9. Behandlungsflüssigkeit zur Verwendung in Verfahren nach Anspruch 7, **dadurch gekennzeichnet, daß** sie ein auf dem Fasermaterial haftend anbringbares Bindemittel umfaßt, in welchem Duftstoffe oder eine geruchsneutralisierende Substanz enthaltende Mikrokapseln dispergiert sind.

10. Behandlungsflüssigkeit zur Verwendung in Verfahren nach Anspruch 9, **dadurch gekennzeichnet, daß** sie zusammen mit einem sich mit ihr nicht vermischenden Treibgas in einem Behälter eingeschlossen ist.
